# EUROPÄISCHE PATENTANMELDUNG

(11) **EP 1 832 568 A1**
(43) Veröffentlichungstag der Anmeldung: **12.09.2007**
(21) Anmeldenummer: 07003977.1
(22) Anmeldetag: 27.02.2007
(51) Int. Cl.: C07C 41/22, C07C 43/225

(54) **Verfahren zur Herstellung von Chlor-1,4-Dimethoxybenzol**

(30) Priorität: 09.03.2006 DE 102006010923
(71) Anmelder: Saltigo GmbH, 51369 Leverkusen (DE)
(72) Erfinder: Schneider, Marielouise, Dr., 51373 Leverkusen (DE); Rodefeld, Lars, Dr., 51375 Leverkusen (DE)
(74) Vertreter: Pettrich, Klaus-Günter

(57) **Zusammenfassung**

Chlor-1,4-dimethoxybenzol wird hergestellt, indem Hydrochinondimethylether mit elementarem Chlor unter der Katalyse von Titantetrachlorid umgesetzt wird. Die erhaltene Rohmischung ist frei an Hydroxyanisol und arm an höherchlorierten Derivaten, so dass sie ohne großen Aufwand zu hochreinem Chlor-1,4-dimethoxybenzol aufdestilliert werden kann.

## Beschreibung

Die vorliegende Erfindung betrifft ein Verfahren zur Herstellung von Chlor-1,4-dimethoxybenzol aus Hydrochinondimethylether.

Chlorierte Hydrochinondimethylether sind bekannte wertvolle Zwischenprodukte für die Herstellung von Farbstoffen, Schädlingsbekämpfungsmitteln und Pharmazeutika. Die Hydrochinondimethylether werden ihrerseits nach bekannten Verfahren durchweg aus den entsprechenden Hydrochinonausgangsverbindungen gewonnen.

So erhält man beispielsweise das technisch bedeutungsvolle Chlor-1,4-dimethoxy-benzol durch direkte Chlorierung von Hydrochinondimethylether (H.E. Akerman, J. Appl. Chem. 3, 416, 1953). Allerdings ist dieses Verfahren immer begleitet von der Bildung größerer Mengen des Disubstitutionsproduktes 2,5-Dichlorhydrochinon-dimethylethers sowie Isomere dieses Disubstitutionsproduktes. Weiterhin wird partielle Etherspaltung zum 4-Hydroxyanisol und dem chlorierten Derivat davon beobachtet. Die Trennung des gewünschten Chlor-1,4-dimethoxybenzol von den beschriebenen unerwünschten Nebenkomponenten ist schwierig und erfordert aufwendige Destillationsverfahren. Bei der Verwendung von Lewis-Säurekatalysatoren wie z.B. Eisen(III)chlorid oder Aluminium(III)chlorid ist die Bildung der Disubstitutionsprodukte zwar etwas vermindert, die Ausbeute an dem gewünschten Chlor-1,4-dimethoxybenzol wird aber durch die Bildung höhermolekularer Verbindungen (z.B. durch unerwünschte Friedel-Crafts-Alkylierungen) unter den Reaktionsbedingungen zusätzlich verringert.

Die Verwendung des Chlorierungsmittels Sulfurylchlorid anstelle von Chlor (Houben-Weyl, Methoden der Organischen Chemie, 4. Auflage, Band 5, 3. S. 883-884, Georg Thieme Verlag, Stuttgart 1962) erbringt keine wesentliche Verbesserung.

Eine neuere Literaturvorschrift (Chemistry Letters, 32(10), 932-933, 2003) beschreibt die Verwendung von *N*-Chlorsuccinimid anstelle von elementarem Chlor in Gegenwart von Ammoniumnitrat oder Eisen(III)chlorid. Benötigt wird hier Acetonitril als Lösungsmittel.

Weitere potentielle Synthesewege zu chlorierten Hydrochinondimethylethem bieten sich durch den Einsatz einer Mischung von Wasserstoffperoxid bzw. *tert*.-Butylwasserstoffperoxid und Chlorwasserstoff (Tetrahedron Letters, 39(35), 6349-6350, 1998), durch Benzyltrimethylammoniumtetrachlorojodat in Eisessig *(*Chemistry Letters, (3), 415-18, 1989) oder durch N-Chlorammoniumsalze in Trifluoressigsäure (Tetrahedron Letters, 24(39), 3117-20, 1983).

Für den technischen Maßstab sind diese Verfahren wenig geeignet, weil in allen Fällen teure und/oder empfindliche Reagenzien und Lösungsmittel zum Einsatz kommen.

Der Erfindung lag daher die Aufgabe zugrunde, ein Verfahren zur Herstellung von Chlor-1,4-dimethoxybenzol aufzufinden, welches ohne besonderen Aufwand, ausgehend von einfach zugänglichen, günstigen Ausgangsprodukten, sicher im technischen Maßstab durchführbar und in Hinblick auf die Nebenkomponenten hochselektiv ist.

Gegenstand der Erfindung ist daher ein Verfahren zur Herstellung von Chkor-1,4-dimethoxybenzol der Formel (I) 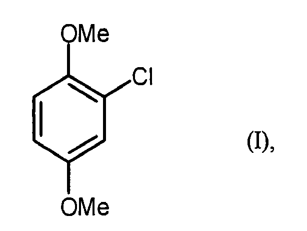 das dadurch gekennzeichnet ist, dass man Hydrochinondimethylether der Formel (II) 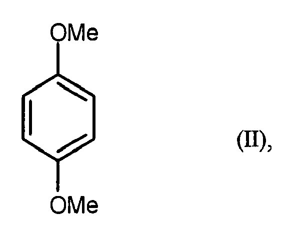 in Substanz oder in einem organischen Lösungsmittel mit elementarem Chlor in Gegenwart von katalytischen Mengen Titantetrachlorid umsetzt.

Dieses erfindungsgemäße Verfahren erfüllt die genannten Anforderungen der gestellten Aufgabe.

Die Umsetzung kann beispielsweise so erfolgen, dass der Hydrochonindimethylether bei 55°C lösungsmittelfrei aufgeschmolzen wird, das Titantetrachlorid zugegeben und anschließend Chlor eingeleitet wird.

Die Reaktionsführung kann weiterhin so erfolgen, dass man den Hydrochinondimethylether in einem inerten organischen Lösungsmittel anlöst, das Titantetrachlorid zugibt und anschließend Chlor einleitet. Als Lösungsmittel kommen beispielsweise halogenierte Kohlenwasserstoffe wie Chlorbenzol, Dichlorbenzole und Trichlorbenzole, halogenierte Benzotrifluoride wie 4-Chlorbenzotrifluorid und Phosphoroxychlorid in Frage.

Die Menge des eingeleiteten Chlors kann pro Mol eingesetztem Hydrochinondimethylethers 0,2 bis 1,0 Äquivalente Chlor betragen. Vorzugsweise führt man der Eduktmischung 0,3 bis 0,5 Mol Chlor pro Mol Hydrochinondimethylether in die Reaktionsmischung zu, besonders vorteilhaft ist die Verwendung von 0,4 Äquivalenten Chlor.

Die Reaktionstemperatur kann beispielsweise 60°C betragen und im Laufe der Reaktion auf 40°C abgesenkt werden. Vorzugsweise wird während der Chlorierung leicht gegen gekühlt, um ein Ansteigen der Temperatur auf > 60°C durch die entstehende Reaktionswärme zu vermeiden.

Die Menge des eingesetzten Titantetrachlorids beträgt üblicherweise 0,1 bis 1 mol-%, vorzugsweise 0,4 bis 0,8 mol pro Mol Hydrochinondimethylether.

Das nach Reaktionsdurchführung vorliegende Gemisch kann man aufarbeiten, indem man zunächst noch vorhandenes Chlor entfernt, z.B. durch Einleiten eines Inertgases oder Anlegen von Vakuum. Das dann vorliegende Rohprodukt kann direkt in die Destillation eingesetzt werden.

Eine weitere Aufarbeitungsvariante des erhaltenen Rohgemisches besteht darin, dass man die Mischung auf 50°C warme verdünnte Salzsäure gibt, die Phasen trennt und die organische Phase dreimal mit 1 %iger Natronlauge wäscht. Anschließend wird die erhaltene Mischung aus Hydrochinondimethylether und Chlor-1,4-dimethoxybenzol aufdestilliert.

Das Verfahren kann großtechnisch so durchgeführt werden, dass der nicht umgesetzte Hydrochinondimethylether nach destillativer Trennung vom Chlor-1,4-dimethoxybenzol in die nächste Partie rückgeführt wird. Die Destillation kann dabei im Kontibetrieb durchgeführt werden.

Mit dem erfindungsgemäßen Verfahren lässt sich Chlor-1,4-dimethoxybenzol aus dem gut zugänglichen Hydrochinondimethylether in einem technisch gut und einfach durchzuführenden Verfahren in guten Ausbeuten und Reinheiten herstellen. Die bei Verwendung von 0,39 Äquivalenten Chlor und 0,6 mol-% Titantetrachlorid nach wässriger Aufarbeitung erhaltene Rohmischung enthält neben 58,6 % nicht umgesetztem Hydrochinondimethylether 38,7 % Chlor-1,4-dimethoxybenzol, 1,7 % 2,5-Dichlorhydrochinondimethylether und 0,03 % (Chlor)-Hydroxyanisol. Aus dieser Mischung lässt sich das Chlor-1,4-dimethoxybenzol ohne großen destillativen Aufwand in > 99,7 %iger Reinheit (bestimmt per GC) und einer Verbrauchszahl von 0,9 (unter Rückführung des nicht umgesetzten Hydrochinondimethylethers) isolieren.

Das bei Verwendung von 0,39 Äquivalenten Chlor nach direkter Destillation ohne vorherige wässrige Aufarbeitung erhaltene Chlor-1,4-dimethoxybenzol wird ebenfalls ohne großen Aufwand in exzellenter Reinheit erhalten.

Somit ist das erfindungsgemäße Verfahren sowohl hinsichtlich der Einfachheit der technischen Durchführung als auch in Hinblick auf Reinheit, Ausbeute und Kosten des erhaltenen Produktes Chlor-1,4-dimethoxybenzols den bisher bekannten Verfahren überlegen.

### Beispiele

### Beispiel 1

### Umsetzung von Hydrochinondimethylether zum Chlor-1,4-dimethoxybenzol unter der Katalyse von Titantetrachlorid

1000 g (7,2 mol) Hydrochinondimethylether wurden im Dreihalskolben als Feststoff vorgelegt und bei 55°C aufgeschmolzen. Zu der Schmelze wurden anschließend 8,3 g (0,6 mol-%) Titantetrachlorid zugegeben, die Heizung entfernt und die Rührergeschwindigkeit auf 130 U/min reguliert. Dann wurde innerhalb von 3 h die berechnete Menge Chlor (200 g, 2,8 mol, 0,39 eq.) in die Reaktionsmischung eingeleitet. Dabei beobachtete man keinen Chlordurchschlag. Die Innentemperatur wurde während der ganzen Reaktionszeit durch leichtes Gegenkühlen auf 50°C gehalten. Nach beendetem Einleiten setzte sich die Reaktionsmischung wie folgt zusammen (GC, norm-%): 58,.6 % Hydrochinondimethylether, 38,7 % Chlor-1,4-dimethoxybenzol, 0,03 % Hydroxyanisol, 0,0 % Chlorhydroxyanisol und 1,7 % 2,5-Dichlorhydrochinondimethylether. Man wusch die Reaktionslösung bei 50-60°C einmal mit 450 ml Wasser + 10 ml HCl_{conc.} und dreimal mit jeweils 450 ml 1 %iger NaOH-Lösung. Die so erhaltene Rohmischung (1092 g) wurde an einer Destillationskolonne mit 15 theoretischen Böden bei 25 bis 30 mbar zu Chlor-1,4-dimethoxybenzol in > 99,7 %iger Reinheit aufdestilliert.

### Vergleichsbeispiel 1

### Unkatalysierte Umsetzung von Hydrochinondimethylether zum Chlor-1,4-dimethoxybenzol

200 g (1,45 mol) Hydrochinondimethylether wurden im Dreihalskolben als Feststoff vorgelegt und bei 55°C aufgeschmolzen. Anschließend wurde die Heizung entfernt und die Rührergeschwindigkeit auf 130 U/min reguliert. Dann wurde innerhalb von 3 h die berechnete Menge Chlor (40 g, 0,56 mol, 0,39 eq.) in die Reaktionsmischung eingeleitet. Dabei beobachtete man keinen Chlordurchschlag. Die Innentemperatur wurde während der ganzen Reaktionszeit durch leichtes Gegenkühlen auf 50°C gehalten. Nach beendetem Einleiten setzte sich die Reaktionsmischung wie folgt zusammen (GC, norm-%): 66,5 % Hydrochinondimethylether, 25,3 % Chlor-1,4-dimethoxybenzol, 0,05 % Hydroxyanisol, 1,26 % Chlorhydroxyanisol und 3 % 2,5-Dichlorhydrochinondimethylether. Um diese Mischung auf eine Reinheit an Chlor-1,4-dimethoxybenzol > 99,7 % aufzudestillieren war eine Destillationskolonne mit einer Trennleistung von 50-100 theoretischen Böden erforderlich.

### Vergleichsbeispiel 2

### Umsetzung von Hydrochinondimethylether zum Chlor-1,4-dimethoxybenzol unter der Katalyse von Eisen(III)chlorid

120 g (0,87 mol) Hydrochinondimethylether wurden im Dreihalskolben als Feststoff vorgelegt und bei 55°C aufgeschmolzen. Zu der Schmelze wurden anschließend 0,93 g (0,66 mol-%) Eisen(III)chlorid zugegeben, die Heizung entfernt und die Rührergeschwindigkeit auf 130 U/min reguliert. Dann wurde innerhalb von 3 h die berechnete Menge Chlor (24 g, 0,34 mol, 0,39 eq.) in die Reaktionsmischung eingeleitet. Dabei beobachtete man keinen Chlordurchschlag. Die Innentemperatur wurde während der ganzen Reaktionszeit durch leichtes Gegenkühlen auf 50°C gehalten. Nach beendetem Einleiten setzte sich die Reaktionsmischung wie folgt zusammen (GC, norm-%): 66,3 % Hydrochinondimethylether, 27,5 % Chlor-1,4-dimethoxybenzol, 3,6 % Hydroxyanisol, 0,4 % Chlorhydroxyanisol und 1,0 % 2,5-Dichlorhydrochinondimethylether. Um diese Mischung auf eine Reinheit an Chlor-1,4-dimethoxybenzol > 99,7 % aufzudestillieren war eine Destillationskolonne mit einer Trennleistung von 50-100 theoretischen Böden erforderlich. Insbesondere die Abtrennung vom Hydroxyanisol erforderte aufgrund des nahezu identischen Siedepunktes zum Chlor-1,4-dimethoxybenzol ein großes Rücklaufverhältnis, was die technische Anwendung des eisen(III)chloridkatalysierten Verfahrens unwirtschaftlich macht.

## Patentansprüche

1. Verfahren zur Herstellung von Chlor-1,4-dimethoxybenzol der Formel (I) 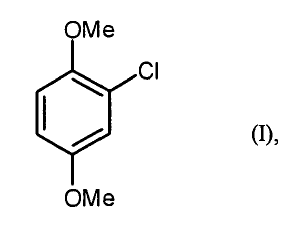 **dadurch gekennzeichnet ist, dass** man Hydrochinondimethylether der Formel (II) 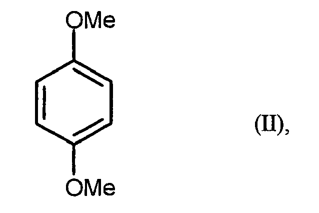 mit elementarem Chlor in Gegenwart von katalytischen Mengen Titantetrachlorid umsetzt.

2. Verfahren nach Anspruch 1, **dadurch gekennzeichnet, dass** man die Reaktion lösungsmittelfrei in einer Schmelze aus Hydrochinondimethylether durchführt.

3. Verfahren nach Anspruch 1, **dadurch gekennzeichnet, dass** man die Reaktion in einem inerten organischen Lösungsmittel durchführt.

4. Verfahren nach Ansprüchen 1 bis 3, **dadurch gekennzeichnet, dass** man 0,1 bis 1 mol-% Titantetrachlorid pro Mol Hydrochinondimethylether einsetzt.

5. Verfahren nach Ansprüchen 1 bis 4, **dadurch gekennzeichnet, dass** man 0,2 bis 1 Äquivalent Chlor pro Mol Hydrochinondimethylether einsetzt.

6. Verfahren nach Ansprüchen 1 bis 5, **dadurch gekennzeichnet, dass** man die erhaltene Rohmischung aufarbeitet, indem man sie auf 50°C warme verdünnte Salzsäure gibt, die Phasen trennt und die organische Phase dreimal mit 1 %iger Natronlauge wäscht.

7. Verfahren nach Ansprüchen 1 bis 5, **dadurch gekennzeichnet, dass** man die erhaltene Rohmischung ohne vorherige wässrige Aufarbeitung direkt aufdestilliert.
